Europäisches Patentamt

European Patent Office

Office européen des brevets

(19)

(11) **EP 1 543 812 A1**

## (12) DEMANDE DE BREVET EUROPEEN

(43) Date de publication:
**22.06.2005 Bulletin 2005/25**

(51) Int Cl.⁷: **A61K 7/02**, A61K 7/48

(21) Numéro de dépôt: **04292949.7**

(22) Date de dépôt: **10.12.2004**

(84) Etats contractants désignés:
**AT BE BG CH CY CZ DE DK EE ES FI FR GB GR HU IE IS IT LI LT LU MC NL PL PT RO SE SI SK TR**
Etats d'extension désignés:
**AL BA HR LV MK YU**

(30) Priorité: **18.12.2003 FR 0351113**

(71) Demandeur: **L'OREAL**
**75008 Paris (FR)**

(72) Inventeur: **Bonafos, Arnaud**
**75014 Paris (FR)**

(74) Mandataire: **Rasson, Catherine**
**L'OREAL - D.I.P.I.**
**25-29 Quai Aulagnier**
**92600 Asnières (FR)**

## (54) Composition démaquillante

(57) L'invention a pour objet une composition cosmétique démaquillante comprenant, dans un milieu physiologiquement acceptable, un mélange d'huiles comprenant l'isohexadécane, le myristate d'isopropyle et le laurate d'hexyle.

La composition selon l'invention est efficace pour le démaquillage et présente un bon confort oculaire. Elle peut être utilisée notamment pour le démaquillage de la peau et/ou de la zone oculaire, et plus particulièrement pour le démaquillage des produits waterproof et sans transfert.

**Description**

L'invention a pour objet une composition démaquillante contenant trois huiles particulières, et l'utilisation cosmétique de la dite composition pour le démaquillage de la peau du visage ou du corps, et/ou le démaquillage de la zone oculaire, et plus particulièrement pour le démaquillage des produits waterproof et sans transfert.

Le démaquillage de la peau est très important pour le soin du visage. Il doit être le plus performant possible car les résidus gras tels que l'excès de sébum, les restes des produits cosmétiques utilisés quotidiennement et les produits de maquillage, s'accumulent dans les replis cutanés et à la surface de la peau, et ils peuvent obstruer les pores de la peau et provoquer ainsi l'apparition de boutons. Une mauvaise qualité de démaquillage et de nettoyage, et en particulier un mauvais rinçage, sont souvent responsables parmi d'autres facteurs de cause, d'un teint brouillé.

Or, aujourd'hui, les consommatrices demandent que les produits de maquillage aient une tenue de plus en plus longue : mascaras waterproof, fonds de teint longue tenue et sans transfert, rouges à lèvres qui tiennent toute la journée, et, pour enlever ces produits, il faut utiliser des formulations de plus en plus efficaces, qui permettent de bien nettoyer la peau tout en la respectant, c'est-à-dire sans l'agresser.

Les compositions démaquillantes peuvent se présenter sous la forme d'une lotion éventuellement biphasique, d'un lait, d'une crème, d'une huile ou encore d'un gel. Pour faciliter le démaquillage, il est connu par exemple d'utiliser des esters gras comme décrit dans le document EP-A-651990. Ces esters permettent en effet de dissoudre très facilement les salissures lipophiles et le maquillage. Toutefois, on recherche une efficacité de démaquillage toujours plus importante.

Il subsiste donc le besoin de compositions démaquillantes encore plus performantes que celles de l'état de la technique.

Or, la demanderesse a découvert de manière surprenante que l'association d'huiles particulières permettait d'obtenir une composition ayant un très bon pouvoir démaquillant, même pour le démaquillage des produits de maquillage waterproof et sans transfert.

La présente invention a donc pour objet une composition cosmétique démaquillante comprenant, dans un milieu physiologiquement acceptable, un mélange d'huiles comprenant l'isohexadécane, le myristate d'isopropyle et le laurate d'hexyle.

Le test de démaquillage décrit ci-après montre que des compositions contenant d'autres composés analogues mais différents n'ont pas une aussi bonne efficacité de démaquillage.

En outre, la composition selon l'invention présente l'avantage d'être douce, d'être bien tolérée et d'être agréable à utiliser car elle ne donne pas de sensation de gras malgré les huiles présentes.

L'invention a également pour objet un procédé cosmétique de démaquillage des matières kératiniques comportant un maquillage, notamment un maquillage waterproof ou sans transfert, consistant à appliquer sur les matières kératiniques, une composition telle que définie précédemment.

L'invention a encore pour objet l'utilisation cosmétique d'une composition cosmétique telle que définie précédemment, pour le démaquillage de la peau et/ou de la zone oculaire, en particulier pour le démaquillage des produits waterproof et sans transfert.

La composition selon l'invention est destinée à une application topique et contient donc un milieu physiologiquement acceptable. Par milieu physiologiquement acceptable, on entend un milieu compatible avec les matières kératiniques, c'est-à-dire la peau, les muqueuses (y compris l'intérieur des paupières et les lèvres), les ongles et/ou les fibres kératiniques (cheveux et cils).

La quantité du mélange d'isohexadécane, de myristate d'isopropyle et de laurate d'hexyle doit être suffisante pour atteindre le but recherché, c'est-à-dire un démaquillage efficace. Cette quantité peut aller par exemple de 5 à 100 % en poids, et de préférence de 10 à 100 % en poids par rapport au poids total de la composition. Chacune de ces huiles doit être présente mais leur quantité respective n'est pas déterminante. De préférence, elles sont présentes en des quantités sensiblement égales, c'est-à-dire avec une différence ne dépassant pas 60 % entre les quantités de chacune des huiles. De préférence, chaque huile peut être présente en une quantité allant de 1 à 40 % en poids par rapport au poids total de la composition.

La composition selon l'invention peut comprendre, outre ces huiles, une ou plusieurs autres huiles choisies parmi les huiles hydrocarbonées (autres que l'isohexadecane), les esters d'acide gras (autres que le myristate d'isopropyle et le laurate d'hexyle), les huiles de silicone, les huiles d'origine végétale, et leurs mélanges.

On entend ici par « huile hydrocarbonée » toute huile comportant majoritairement des atomes de carbone et d'hydrogène. Comme huiles hydrocarbonées, on peut citer par exemple les huiles de paraffine, volatiles ou non, et leurs dérivés, l'huile de vaseline, les polydécènes, le polyisobutène hydrogéné tel que l'huile de

Parléam®, l'isoparaffine hydrogénée, l'isododecane, et leurs mélanges.

Les esters d'acide gras pouvant être utilisés dans la composition de l'invention comportent au moins 8 atomes de carbone, de préférence de 9 à 26 atomes de carbone et mieux de 12 à 22 atomes de carbone. Les esters sont de préférence de mono- ou di-esters. De manière préférée, les esters ne comportent aucune insaturation et/ou aucun groupement éther ou hydroxyle. De façon encore plus avantageuse, il s'agit d'un ester saturé qui ne renferme aucun groupement éther ni hydroxyle. Les esters d'acide gras peuvent être notamment choisis dans le groupe comprenant le palmitate d'éthyl-2 hexyle (ou palmitate d'octyle), l'isononanoate d'isononyle, le lactate d'isostéaryle, le myristate d'éthyl-2 hexyle (ou myristate d'octyle), le palmitate d'isopropyle, l'adipate de di-isopropyle, l'adipate de di-octyle, l'hexanoate d'éthyl-2 hexyle, le laurate d'éthyle, le myristate de méthyle, l'octanoate d'octyldodécyle, le néopentanoate d'isodécyle, le myristate d'éthyle, le propionate de myristyle, l'éthyl-2 hexanoate d'éthyl-2 hexyle, l'octanoate d'éthyl-2 hexyle, le caprate/caprylate d'éthyl-2 hexyle, le palmitate de méthyle, le myristate de butyle, le myristate d'isobutyle, le palmitate d'éthyle, le laurate d'isohexyle, l'isostéarate d'isopropyle, l'ester d'acide benzoïque et d'alcools en $C_{12}$-$C_{15}$ (nom CTFA: C12-15 Alkyl Benzoate), le coco-caprylate/caprate ; et leurs mélanges.

Comme huiles d'origine végétale, on peut citer par exemple l'huile d'amande douce, l'huile d'avocat, l'huile de cassis, l'huile de bourrache, l'huile de chanvre, l'huile de ricin, l'huile de coriandre, l'huile d'olive, l'huile de jojoba, l'huile de sésame, l'huile d'arachide, l'huile de pépins de raisin, l'huile de colza, l'huile de coprah, l'huile de noisette, le beurre de karité, l'huile de palme, l'huile de noyau d'abricot, l'huile de calophyllum, l'huile de son de riz, l'huile de germes de maïs, l'huile de germes de blé, l'huile de soja, l'huile de tournesol, l'huile d'onagre, l'huile de carthame, l'huile de passiflore, l'huile de seigle, les triglycérides des acides caprylique/caprique comme ceux vendus par la société Stearineries Dubois ou ceux vendus sous les dénominations Miglyol 810, 812 et 818 par la société Dynamit Nobel ;

Comme huiles de silicone, on peut citer par exemple les polyméthylsiloxanes (PDMS) volatiles ou non à chaîne siliconée linéaire ou cyclique, liquides ou pâteux à température ambiante, notamment les cyclopolydiméthylsiloxanes (cyclométhicones) telles que la cyclohexasiloxane et la cyclopentasiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényl-diméthicones, les diphénylméthyldiphényl trisiloxanes, les 2-phényléthyltriméthyl-siloxysilicates, et les polyméthylphénylsiloxanes, leurs mélanges.

La composition selon l'invention peut se présenter sous forme d'émulsion, de biphase ou de composition anhydre.

Dans la présente demande, on entend par « composition anhydre », une composition qui contient moins de 2 % en poids d'eau, voire moins de 0,5 % d'eau par rapport au poids total de la composition, et notamment une composition exempte d'eau, l'eau n'étant pas ajoutée lors de la préparation de la composition mais correspondant à l'eau résiduelle apportée par les ingrédients mélangés.

Quand la composition selon l'invention est une émulsion ou une composition biphase, elle comporte une phase aqueuse et une phase huileuse. Les émulsions et les compositions biphase se distinguent l'une de l'autre par le fait que les compositions biphases sont constituées de deux phases qui, au repos, sont distinctes au lieu d'être émulsionnées l'une dans l'autre. L'utilisation de ces compositions biphases nécessite une agitation préalable afin de former une émulsion extemporanée, celle-ci devant être de qualité et de stabilité suffisantes pour permettre une application homogène des deux phases, mais telle qu'au repos, lesdites phases se séparent rapidement et retrouvent leur état initial, ce phénomène étant plus connu sous le terme de "déphasage". En revanche, les émulsions comportent deux phases, une phase aqueuse et une phase huileuse, l'une des phases étant dispersée dans l'autre de manière stable.

Quand la composition selon l'invention contient une phase aqueuse, celle-ci contient de l'eau et tout additif hydrosoluble ou hydrodispersible. L'eau utilisée peut être de l'eau pure déminéralisée mais aussi de l'eau minérale et/ou de l'eau thermale et/ou de l'eau de mer, c'est-à-dire que l'eau de la composition peut être en partie ou totalement constituée par une eau choisie parmi les eaux minérales, les eaux thermales, les eaux de mer et leurs mélanges. En général, une eau minérale est propre à la consommation, ce qui n'est pas toujours le cas d'une eau thermale. Chacune de ces eaux contient, entre autre, des minéraux solubilisés et/ou des oligo-éléments. Ces eaux sont connues pour être employées à des fins de traitement spécifique selon les oligo-éléments et les minéraux particuliers qu'elles contiennent, tel que l'hydratation et la désensibilisation de la peau ou le traitement de certaines dermatoses. Par eaux minérales ou thermales, on désignera non seulement les eaux minérales ou thermales naturelles, mais également des eaux minérales ou thermales naturelles enrichies en constituants minéraux et/ou en oligo-éléments supplémentaires, ainsi que des solutions aqueuses minérales et/ou contenant des oligo-éléments préparées à partir d'eau purifiée (déminéralisée ou distillée).

Une eau thermale ou minérale naturelle utilisée selon l'invention peut, par exemple, être choisie parmi l'eau de Vittel, les eaux du bassin de Vichy, l'eau d'Uriage, l'eau de la Roche Posay, l'eau de la Bourboule, l'eau d'Enghien-les-Bains, l'eau de Saint Gervais-les-Bains, l'eau de Néris-les-Bains, l'eau d'Allevar-les-Bains, l'eau de Digne, l'eau de Maizières, l'eau de Neyrac-les-Bains, l'eau de Lons-le-Saunier, les Eaux Bonnes, l'eau de Rochefort, l'eau de Saint Christau, l'eau des Fumades et l'eau de Tercis-les-bains, l'eau d'Avene.

On peut aussi utiliser des eaux de mer telles que l'eau de la mer Morte (Dead Sea water) ou l'eau des fonds marins.

La phase aqueuse peut également comprendre des solvants organiques miscibles à l'eau (à 25 °C) comme par exemple les alcools primaires (alcool monohydrique en $C_1$-$C_3$) tels que l'éthanol et l'isopropanol, les polyols tels que le propylène glycol, le butylène glycol, la glycérine, l'hexylène glycol, les polyéthylène glycols comme le PEG-8, le dipropylène glycol et leurs mélanges. La quantité de polyol(s) dans la composition de l'invention est de préférence telle qu'elle ne confère pas de caractère collant à la composition finale. Cette quantité va en général de 0,05 à 20 % en poids et de préférence de 0,1 à 15 % en poids et mieux de 0,5 à 10 % en poids par rapport au poids total de la composition.

Quand la composition se présente sous forme d'une émulsion, il peut s'agir d'une émulsion eau-dans-huile (E/H) ou huile-dans-eau (H/E), ou d'une émulsion multiple (E/H/E ou H/E/H). Le rapport pondéral entre la phase aqueuse et la phase huileuse peut être par exemple de 10/90 à 95/5, et de préférence de 30/70 à 90/10. Ainsi, la phase aqueuse peut représenter par exemple de 10 à 99 % en poids, de préférence de 30 à 98 % en poids, mieux de 60 à 95 % en poids par rapport au poids total de la composition, et la phase huileuse peut représenter par exemple de 1 à 90 % en poids, de préférence de 2 à 70 % en poids, mieux de 5 à 40 % en poids par rapport au poids total de la composition.

Quand la composition se présente sous forme d'un biphase, le rapport pondéral entre la phase aqueuse et la phase huileuse va de préférence, de 25/75 à 90/10 et de préférence 30/70 à 70/30. La phase aqueuse représente donc généralement de 25 à 90 % en poids, de préférence de 30 à 70 % en poids et mieux de 40 à 60 % en poids par rapport au poids total de la composition.

Selon un mode de réalisation préféré de l'invention, la composition se présente sous forme d'une émulsion H/E et constitue un lait démaquillant.

La composition de l'invention peut comprendre aussi un ou plusieurs tensioactifs. La quantité de tensioactif (s) (en matière active) va de préférence de 0,01 à 20 % en poids, plus préférentiellement 0,05 à 10 % en poids et mieux de 0,05 à 5 % par rapport au poids total de la composition.

On peut utiliser comme tensioactif, tout tensioactif habituellement utilisé dans les compositions démaquillantes. Le tensioactif peut être choisi parmi les tensioactifs non ioniques, les tensioactifs anioniques, les tensioactifs amphotères ou zwitterioniques et leurs mélanges.

On peut citer comme tensioactifs utilisables dans la composition selon l'invention :

(1) parmi les tensioactifs non ioniques :

- les polymères blocs oxyéthylénés oxypropylénés tels que les Poloxamers et notamment le Poloxamer 184 (nom CTFA) ;
- les esters d'acide gras de sorbitan et leurs dérivés oxyéthylénés, comme le monostéarate de sorbitan (nom CTFA : Sorbitan stearate) vendu par la société ICI sous la dénomination Span 60, le monopalmitate de sorbitan (nom CTFA : Sorbitan palmitate) vendu par la société ICI sous la dénomination Span 40, les stéarates, palmitates et oléates de sorbitan oxyéthylénés (nom CTFA : Polysorbate) vendus par la société ICI sous les dénominations Tween, notamment le Polysorbate 60 (Tween 60), le Polysorbate 65 (Tween 65), le Polysorbate 80 (Tween 80);
- les éthers oxyéthylénés de dihydrocholestérol, tels que le Dihydrocholeth-15, le Dihydrocholeth-20, le Dihydrocholeth-30 ;
- les alkylpolyglycosides et notamment les alkylpolyglucosides (APG) ayant un groupe alkyle comportant de 6 à 30 atomes de carbone (alkyl-$C_6$-$C_{30}$ polyglucosides) et de préférence 8 à 16 atomes de carbone, comme par exemple le decylglucoside (Alkyl-C9/C11-polyglucoside (1.4) tel que le produit commercialisé sous la dénomination MYDOL 10 par la société Kao Chemicals, le produit commercialisé sous la dénomination PLANTAREN 2000 UP ou PLANTACARE 2000 UP par la société Cognis, et le produit commercialisé sous la dénomination ORAMIX NS 10 par la société Seppic ; le caprylyl/capryl glucoside comme le produit commercialisé sous la dénomination ORAMIX CG 110 par la Société Seppic ; le laurylglucoside comme les produits commercialisés sous les dénominations PLANTAREN 1200 N et PLANTACARE 1200 par la société Cognis ; et le coco-glucoside comme le produit commercialisé sous la dénomination PLANTACARE 818/UP par la société Cognis,
- les dérivés oxyéthylénés d'huile hydrogénée, tels que PEG-60 hydrogenated castor oil.

(2) parmi les tensioactifs anioniques :

- les alkylsulfates et leurs sels, les alkyl éther sulfates et leurs sels, notamment leurs sels de sodium, comme le mélange de Sodium Laureth Sulfate /Magnesium Laureth Sulfate / Sodium Laureth-8 Sulfate / Magnesium Laureth-8 Sulfate, vendu sous le nom de Texapon ASV par la société Cognis ; le lauryl éther sulfate de sodium (C12-14 70/30) (2,2 OE) commercialisé sous les dénominations SIPON AOS 225 ou Texapon N702 PATE par la société Cognis ; le lauryl éther sulfate d'ammonium (C12-14 70/30) (3 OE) commercialisé sous la dénomination SIPON LEA 370 par la société Cognis ; l'alkyl (C12-C14) éther (9 OE) sulfate d'ammonium commercialisé sous la dénomination RHODAPEX AB/20 par la société Rhodia Chimie.

(3) parmi les tensioactifs amphotères ou zwitterioniques, les alkylamphoacétates tels que le N-cocoyl-N-carboxyméthoxyéthyl-N-carboxyméthyl-éthylènediamine N-di-sodique (nom CTFA : Disodium cocoamphodiacetate) commercialisé en solution aqueuse saline sous la dénomination MIRANOL C2M CONC NP par la société Rhodia Chimie ; le N-cocoyl-N-hydroxyéthyl-N-carboxyméthyl-éthylènediamine N-sodique (nom CTFA: sodium cocamphoacetate) ; les cocamides tels que le mélange d'éthanolamides d'acide de coco (nom CTFA : Cocamide DEA).

La composition peut comprendre aussi un mélange de deux ou plus de ces tensioactifs.

La composition selon l'invention peut également contenir des adjuvants ou additifs cosmétiques conventionnels qui se trouveront dans l'une ou l'autre phase selon leur nature hydrophile ou lipophile, tels que par exemple des actifs, des parfums, des agents conservateurs et bactéricides, des colorants, des agents adoucissants, des tampons, des humectants, des filtres U.V. (ou filtres solaires), des électrolytes tel que le chlorure de sodium ou un ajusteur de pH par exemple acide citrique ou hydroxyde de sodium), et leurs mélanges. Bien entendu l'homme du métier veillera à choisir ce ou ces éventuels additifs et/ou leur quantité, de manière telle que les propriétés avantageuses de la composition selon l'invention ne soient pas, ou substantiellement pas, altérées par l'adjonction envisagée.

Comme conservateurs, on peut utiliser tout conservateur habituellement utilisé dans les domaines considérés, tels que par exemple les parabens ; le digluconate de chlorhexidine ; un bromure d'alkyl triméthylammonium tel que le bromure de myristyl-triméthylammonium (nom CTFA : Myrtrimonium bromide), le bromure de dodécyl-triméthylammonium, le bromure d'hexadécyl-triméthylammonium, et leurs mélanges tel que le mélange vendu sous la dénomination Cetrimide par la société FEF CHEMICALS ; les sels d'ammonium quaternaires de polymère, tels que le polyquaternium -10.

Comme bactéricide, on peut par exemple utiliser un mono($C_3$-$C_9$)alkyl-ou ($C_3$-$C_9$)alcényléther de glycérol dont la fabrication est décrite dans la littérature, en particulier dans E. Baer, H.O.L. Fischer - J. Biol. Chem. 140-397-1941. Parmi ces mono($C_3$-$C_9$)alkyl-ou ($C_3$-$C_9$)alcényléthers de glycérol, on utilise de préférence le 3-[(2-ethylhexyl)oxy]-1,2-propanediol, le 3-[(heptyl)oxy]-1,2-propanediol, le 3-[(octyl)oxy]-1,2-propanediol et le 3-[(allyl)oxy]-1,2-propanediol. Un mono($C_3$-$C_9$)alkyléther de glycérol plus particulièrement préféré selon la présente invention est le 3-[(2-ethylhexyl)oxy]-1,2-propanediol, vendu par la société SCHULKE & MAYR G.m.b.H. sous la dénomination commerciale SENSIVA SC 50 (nom INCI : Ethylhexylglycerin).

Comme actifs utilisables dans la composition de l'invention, on peut citer par exemple les agents apaisants comme l'allantoïne et le bisabolol ; les eaux florales telles que l'eau de tilleul ou l'eau de bleuet ; l'acide glycyrrhétinique et ses sels ; les antibactériens comme l'octopirox, le triclosan et le triclocarban ; les huiles essentielles ; les vitamines telles que par exemple le rétinol (vitamine A), l'acide ascorbique (vitamine C), le tocophérol (vitamine E), la niacinamide (vitamine PP ou B3), le panthénol (vitamine B5) et leurs dérivés tels que par exemple les esters de ces vitamines (palmitate, acétate, propionate), l'ascorbyl phosphate de magnésium, la vitamine C glycosylée ou acide glucopyranosyl ascorbique (Ascorbyl glucoside) ; les co-enzymes tels que le co-enzyme Q10 ou ubiquinone et le co-enzyme R ou biotine ; les hydrolysats de protéine ; les extraits végétaux et les extraits de plancton ; et leurs mélanges.

Quand la composition de démaquillage est un biphase, elle peut contenir aussi un agent de déphasage, qui permet d'améliorer la vitesse de séparation des phase après agitation. Comme agent de déphasage, on peut citer par exemple les chlorures d'alkyldiméthylbenzylammonium comme décrit dans le document EP-A-603080, et notamment le chlorure de benzalkonium, et les mélanges le contenant ; les alkyl glucosides alcoxylés comportant un groupe d'ammonium quaternaire et notamment le chlorure de laurylmethylgluceth-10 hydroxypropyldimonium, comme décrit dans le document EP-A-847746 ; les polymères et copolymères de vinylpyrrolidone et notamment le copolymère de polyvinylpyrrolidone / hexadecene comme décrit dans le document WO-A-99/56704 ; et leurs mélanges. Quand un tel agent est présent, le rapport entre l'agent tensioactif et l'agent de déphasage va de préférence de 0,005/1 à 200/1 et mieux de 0,01/1 à 120/1. L'agent de déphasage peut être présent en une proportion allant par exemple de 0,025 à 5 % en poids par rapport au poids total de la composition.

L'exemple suivant est donné à titre d'illustration de l'invention et n'a pas de caractère limitatif. Toutes les quantités sont données en pourcentage en poids de matière première par rapport au poids total de la composition.

Exemple selon l'invention : Composition démaquillante (Emulsion H/E)

| Phase A | |
|---|---|
| Disodium cocamphoacétate (tensioactif) | 0,1 % |
| Glycérine | 3 % |
| Methylparaben | 0,12 % |
| Mytrimonium bromide | 0,025 % |
| Carbomer | 0,6 % |
| PEG-60 Hydrogenated Castor Oil | 0,6 % |
| Eau | qsp 100 % |

| Phase B | |
|---|---|
| Propylparaben | 0,02 % |
| Myristate d'isopropyle | 8 % |
| Laurate d'hexyle (Cetiol A de la société Cognis) | 5 % |
| Isohexadecane | 5 % |

| Phase C | |
|---|---|
| Hydroxyde de sodium | 0,24 % |
| Eau | 5 % |

Mode opératoire : On prépare à chaud les phases aqueuses et huileuses, et on fait l'émulsion en versant la phase huileuse dans la phase aqueuse, puis on neutralise par la phase C et on refroidit.
On obtient une composition démaquillante efficace pour le démaquillage et agréable à utiliser car très douce et ne donnant pas de sensation de gras.
Exemples comparatifs :

- Exemple comparatif 1 : on remplace dans l'exemple selon l'invention, 5 % de laurate d'hexyle par 5 % d'isononanoate d'isononyle.
- Exemple comparatif 2 : on remplace dans l'exemple selon l'invention, 5 % de laurate d'hexyle par 5 % de coco-caprylate/caprate (Cetiol LC de la société Cognis).
- Exemple comparatif 3 : on remplace dans l'exemple selon l'invention, 5 % de laurate d'hexyle par 5 % de palmitate d'éthyle et on ajoute 3 % supplémentaire de myristate d'isopropyle.
- Exemple comparatif 4 : on remplace dans l'exemple selon l'invention, 5 % de myristate d'isopropyle et 5 % d'isohexadecane par 5 % de coco-caprylate/caprate et par 5 % d'isononanoate d'isononyle.
- Exemple comparatif 5 : on remplace dans l'exemple selon l'invention, 5 % de myristate d'isopropyle et 5 % de laurate d'hexyle par 5 % de coco-caprylate/caprate et par 5 % d'isononanoate d'isononyle.
- Exemple comparatif 6 : on remplace dans l'exemple selon l'invention, 5 % de myristate d'isopropyle par 5 % de coco-caprylate/caprate.

Mesure du pouvoir démaquillant

Le pouvoir démaquillant de la composition selon l'invention et des exemples comparatifs a été déterminé selon le protocole décrit ci-dessous.

1. Matériel

- Composition démaquillante
- Savon de Marseille
- 1 cache découpé dans une fiche bristol de 4x4 cm + 1 feutre LUMICOLOR permanent S

- Colorimètre CR300 (mesure de la colorimétrie en L.a.b)
- Fond de teint sans transfert Air Wear LSF 12
- 8 petits verres de montre + des petites spatules
- 1 balance de précision
- 1 chronomètre

2. Protocole

a- Tracer sur la peau des bras, 4 zones (2 par bras) à l'aide du feutre et du cache
b- Nettoyer la peau au savon, rincer et sécher (les marques de feutre sont alors atténuées pour éviter tout transfert de feutre dans le fond de teint au moment de l'application).
c-Mesurer par colorimétrie la peau nue : 3 mesures de L,a,b par zone
d- Appliquer dans les 4 zones délimitées, de façon homogène, 16mg+/-0,05mg de fond de teint et attendre 30 minutes
e- Mesurer par colorimétrie la peau avec fond de teint : 3 mesures de L,a,b par zone
f- Peser dans les verres de montre 4 x 208 mg+/-1 mg de produit démaquillant à tester
g- Démaquiller la zone au doigt par mouvement circulaire pendant 10 secondes puis rincer à l'eau du robinet (tiède ; dureté non contrôlée) en effleurant légèrement avec les doigts de la main disponible. Faire les 4 zones sans attendre.
h- Tamponner avec un mouchoir en papier type kleenex et attendre 15 minutes (sensation de peau sèche)
i- Mesurer par colorimétrie la peau démaquillée : : 3 mesures de L,a,b par zone

3. Calcul du pourcentage de démaquillage

a- Ecart colorimétrique de la peau maquillée par rapport à la peau nue = RA $\Delta$Emax :

$$\Delta E \text{ max} = \surd \ (\Delta a_1{}^2 + \Delta b_1{}^2 + \Delta L_1{}^2)$$

avec

$\Delta a_1$ = a peau nue - a peau FdT
$\Delta b_1$ = b peau nue - b peau FdT
$\Delta L_1$ = L peau nue - L peau FdT

b- Pour un produit démaquillant : Ecart colorimétrique de la peau démaquillée par rapport à la peau maquillée = $\Delta$E pour chaque zone : $\Delta E = (\Delta a_2{}^2 + \Delta b_2{}^2 + \Delta L_2{}^2)$ avec

$\Delta a_2$ = a peau démaq - a peau FdT
$\Delta b_2$ = b peau démaq - b peau FdT
$\Delta L_2$ = L peau démaq - L peau FdT

Le pourcentage de démaquillage moyen correspond à la moyenne des 4 valeurs de ($\Delta E/\Delta Emax$) *100 pour un produit démaquillant donné.
Le tableau ci-dessous donne les résultats du pourcentage de démaquillage (pourcentage de maquillage enlevé) pour chaque composition.

| Composition | Pourcentage de démaquillage |
|---|---|
| Exemple selon l'invention | 68,3 % |
| Exemple comparatif 1 | 39,1 % |
| Exemple comparatif 2 | 34,4 % |
| Exemple comparatif 3 | 40 % |
| Exemple comparatif 4 | 28,5 % |
| Exemple comparatif 5 | 40,3 % |

(suite)

| Composition | Pourcentage de démaquillage |
|---|---|
| Exemple comparatif 6 | 46,8 % |

Ce tableau montre que la composition selon l'invention présente une efficacité de démaquillage bien plus importante que les compositions des exemples comparatifs.

**Revendications**

1.  Composition cosmétique démaquillante comprenant, dans un milieu physiologiquement acceptable, un mélange d'huiles comprenant l'isohexadécane, le myristate d'isopropyle et le laurate d'hexyle.

2.  Composition selon la revendication 1, **caractérisée par le fait que** le mélange est en une quantité allant de 5 à 100 % en poids par rapport au poids total de la composition.

3.  Composition selon la revendication 1 ou 2, **caractérisée par le fait que** chaque huile est présente en une quantité allant de 1 à 40 % en poids par rapport au poids total de la composition.

4.  Composition selon l'une quelconque des revendications précédentes, **caractérisée par le fait qu'**elle comprend en outre, une ou plusieurs autres huiles choisies parmi les huiles hydrocarbonées, les esters d'acide gras, les huiles de silicone, les huiles d'origine végétale, et leurs mélanges.

5.  Composition selon l'une quelconque des revendications précédentes, **caractérisée par le fait qu'**elle contient un ou plusieurs tensioactifs.

6.  Composition selon l'une quelconque des revendications précédentes, **caractérisée par le fait que** la quantité de tensioactif(s) va de 0,01 à 20 % en poids par rapport au poids total de la composition.

7.  Composition selon l'une quelconque des revendications précédentes, **caractérisée par le fait qu'**elle se présente sous forme d'émulsion, de biphase ou de composition anhydre.

8.  Composition selon l'une quelconque des revendications précédentes, **caractérisée par le fait qu'**elle se présente sous forme d'émulsion H/E.

9.  Utilisation cosmétique de la composition cosmétique selon l'une quelconque des revendications 1 à 8, pour le démaquillage de la peau et/ou de la zone oculaire.

10. Procédé cosmétique de démaquillage des matières kératiniques comportant un maquillage, consistant à appliquer sur les matières kératiniques, une composition selon l'une quelconque des revendications 1 à 8.

**Office européen**

**des brevets**

**RAPPORT DE RECHERCHE EUROPEENNE**

Numéro de la demande

EP 04 29 2949

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| Y | EP 1 034 774 A (OREAL) 13 septembre 2000 (2000-09-13) * exemples * * revendications * ----- | 1-10 | A61K7/02 A61K7/48 |
| Y,D | EP 0 651 990 A (OREAL) 10 mai 1995 (1995-05-10) * colonne 2, ligne 7 - ligne 15 * * revendications * ----- | 1-10 | |

**DOMAINES TECHNIQUES RECHERCHES (Int.Cl.7)**

A61K

Le présent rapport a été établi pour toutes les revendications

| Lieu de la recherche | Date d'achèvement de la recherche | Examinateur |
|---|---|---|
| Berlin | 25 février 2005 | Pelli Wablat, B |

EPO FORM 1503 03.82 (P04C02)

**ANNEXE AU RAPPORT DE RECHERCHE EUROPEENNE
RELATIF A LA DEMANDE DE BREVET EUROPEEN NO.**

EP 04 29 2949

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

25-02-2005

| Document brevet cité au rapport de recherche | | Date de publication | Membre(s) de la famille de brevet(s) | | Date de publication |
|---|---|---|---|---|---|
| EP 1034774 | A | 13-09-2000 | FR | 2789307 A1 | 11-08-2000 |
| | | | AT | 217512 T | 15-06-2002 |
| | | | BR | 0000594 A | 02-05-2001 |
| | | | DE | 69901482 D1 | 20-06-2002 |
| | | | DE | 69901482 T2 | 29-08-2002 |
| | | | EP | 1034774 A1 | 13-09-2000 |
| | | | ES | 2177208 T3 | 01-12-2002 |
| | | | JP | 2000229814 A | 22-08-2000 |
| | | | KR | 2000057965 A | 25-09-2000 |
| | | | US | 6342469 B1 | 29-01-2002 |
| EP 0651990 | A | 10-05-1995 | FR | 2711917 A1 | 12-05-1995 |
| | | | AT | 172100 T | 15-10-1998 |
| | | | CA | 2135130 A1 | 06-05-1995 |
| | | | DE | 69413924 D1 | 19-11-1998 |
| | | | DE | 69413924 T2 | 04-03-1999 |
| | | | EP | 0651990 A1 | 10-05-1995 |
| | | | ES | 2125426 T3 | 01-03-1999 |
| | | | JP | 2898209 B2 | 31-05-1999 |
| | | | JP | 7196444 A | 01-08-1995 |

EPO FORM P0460

Pour tout renseignement concernant cette annexe : voir Journal Officiel de l'Office européen des brevets, No.12/82